**Europäisches Patentamt**

**European Patent Office**

**Office européen des brevets**

(19)

(11) Publication number : **0 325 816 B1**

# (12) EUROPEAN PATENT SPECIFICATION

(45) Date of publication of patent specification :
**18.03.92 Bulletin 92/12**

(51) Int. Cl.$^5$ : **A01G 7/00**

(21) Application number : **88203013.3**

(22) Date of filing : **23.12.88**

(54) **Method for forcing hydrangea plants into bloom all year round.**

(30) Priority : **05.01.88 NL 8800010**

(43) Date of publication of application :
**02.08.89 Bulletin 89/31**

(45) Publication of the grant of the patent :
**18.03.92 Bulletin 92/12**

(84) Designated Contracting States :
**AT BE CH DE ES FR GB GR IT LI LU NL SE**

(56) References cited :
**DE-C- 80 284**
**FR-A- 2 032 255**
**NL-A- 8 204 053**
**R.AUGE et al.: "La culture in vitro et ses**
**applications horticoles",1982, Technique &**
**Documentation, Paris, FR, p. 93**

(73) Proprietor : **Eveleens, Leo Anne**
**Poelweg 36**
**NL-1424 PB De Kwakel (NL)**

(72) Inventor : **Eveleens, Leo Anne**
**Poelweg 36**
**NL-1424 PB De Kwakel (NL)**

(74) Representative : **Baarslag, Aldert D. et al**
**Nederlandsch Octrooibureau**
**Scheveningseweg 82 P.O. Box 29720**
**NL-2502 LS 's-Gravenhage (NL)**

## Description

The invention relates to a method for forcing hydrangea plants (<u>Hydrangea macrophylla</u> (Thunb.) Ser.) into bloom all year round after application of a dormant period for the hydrangea plants at reduced temperature.

The hydrangea plant is a semi-shrub and belongs in respect of origin to the prehistoric vegetation of Japan. Although the hydrangea has already been cultivated in Europe for a long time an upgrading process for the hydrangea plant did not come into being until approximately 1900. Originally, the actual flowers of the umbelliform hydrangea flowers were very small and the florets of the ray which act as attracting flowers were large and colourful. In modern varieties virtually the entire flower consists of these sterile attracting flowers, and because of the sterility thereof the hydrangea flower umbels can last for approximately 6 weeks.

The cultivation of hydrangeas can be subdivided into a number of steps:

a) the production of cuttings, female plants normally being used in the case of the traditional cultivation method;

b) the cultivation of the cuttings, which at present growing, usually takes 1 year;

c) the flower bud formation, which by nature mandatorily takes place in Europe from about mid-August to mid-October. This flower bud formation is subsequently accompanied by lignification of the stalk and dying-off of the leaf;

d) the dormant period (cold period) for the hydrangeas provided with a flower bud formation; and

e) breaking-through from the dormant period after which the hydrangeas are forced into bloom.

The cold treatment reported under d) optionally takes place in the cold greenhouse or in a cold store. The advantage of using a cold store resides in the fact that the temperature and relative humidity can be better controlled therein than in a cold greenhouse and that as a result the bloom can be better spread. In fact, it is desirable to spread the bloom over as large a period of the year as possible. The present maximum bloom spreading is achieved by storing the lignified hydrangeas, which are provided with a flower bud formation, in a cold store at 1-2°C (relative humidity: 90%). The bloom of the hydrangeas can be spread from January to June in this way. Storage for a still longer period is difficult because the plants will also develop fully at some time even in the cold store.

It has been found that it is possible to force hydrangeas into bloom all year round when hydrangea plants grown from tissue culture plants are used which have undergone a dormant period at a temperature below 0°C after flower bud formation and lignification.

In connection with the above it is put forward that subjecting hydrangea plants cultivated in the traditional manner to a dormant period at a temperature below 0°C, for example -2°C, leads to particularly great shedding (of, for example, 80% or more), depending on the hydrangea variety. On the basis of this large shedding it can be put that such a negative result in the hydrangea plants cultured in the traditional way gives rise to prejudice with regard to the use of a "freezing temperature" during the dormant period of hydrangea plants so that the positive result, obtained with the method according to the invention, of virtually no shedding in hydrangea plants cultured from tissue culture plants is to be noted as being particularly surprising.

The invention therefore relates to a method for forcing hydrangea plants into bloom all year round which is characterized in that hydrangea plants grown from tissue culture material are used which, after they were completely dormant, have undergone a dormant period at a temperature below 0°C. The hydrangea plants are completely dormant when the stalk is lignified and the flower buds closed.

Hydrangea plants which are grown from tissue culture material and which have undergone a dormant period at a temperature of 0 to -5°C, preferably of -0.5°C to -2°C, after formation of the flower bud and lignification are advantageously used. The above-described dormant period can cover a period of 1-12 months, but is preferably 6-12 months since this period corresponds to the period in which no bloom is possible with the aid of the hydrangea plants cultivated in the traditional way.

Finally, the invention relates to the storage of hydrangea plants which are lignified and possess flower buds during the dormant period at a temperature below 0°C, advantageously at 0 to -5°C, and very particularly at -0.5° to -2°C.

Examples of hydrangea varieties which can be used in the method according to the invention are: Adria (blue, early), Alpenglühn (red, early), Bodensee (pink/blue, middle), King George V (red, middle), Konigstein (red, middle), Masja (pink), Neu Friesdorf (pink, middle), Reinhold Ambrosius (blue, early), Schnee (red, early) and Wildenstein (red, early).

In contrast to the traditional manner of cultivation in which female plants are usually used for the production of cuttings, no separate stock of female plants is required in the cultivation of plants from tissue culture. In principle, the stock kept in tissue culture can serve as "stock of female plants". Only a few plants, which provide the starting material (young shoots or auxillary buds) for the tissue culture, are necessary for culturing such a stock.

The tissue culture according to the invention, i.e. the culturing of hydrangea plants in vitro, can take place in many ways, such as: root formation of isolated buds, regeneration of plants from meristems and shoot tops, regeneration from explants and regeneration from callus tissue. The tissue culture plants to be used as starting material in the method according to the invention have been cultured as follows on the basis of tissue culture material:

Small growing plants were freed from moulds, bacteria and eelworms in a lengthy process, account being taken of the sensitivity of hydrangea material to disinfectants. The plants were then divided with small knives after which the tissue pieces were grown under sterile conditions at an illumination of about 500 Lux per m² for 8 hours per twenty-four hours and at a temperature of 20°C on a nutrient medium having the following composition:

## Composition of nutrient medium
### per litre

| | |
|---|---|
| $NH_4NO_3$ | 1.650 g |
| $KNO_3$ | 1.900 g |
| $CaCl_2x2H_2O$ | 0.440 g |
| $MgSO_4x7H_2O$ | 0.370 g |
| $KH_2PO_4$ | 0.170 g |
| NaFeEDTA | 0.025 g |

| | | |
|---|---|---|
| Inositol | 0.100 g | (Sigma Chemical Company, Saint Louis, USA) |
| Thiamine | 0.003 g | |
| nicotine acid | 0.003 g | |
| Pyridoxine | 0.003 g | |

MS-micro 1/1 10 ml stock 100x

IAA 0.25 mg/l = 0.5 ml of stock $0.5x10^{-3}$ (IAA = indolylacetic acid)

Kin. 0.2 mg/l = 0.2 ml of stock $1.0x10^{-3}$ (Kin = kinetine)

| | |
|---|---|
| Sucrose | 25.000 g |
| Agar | 6.000 g |

pH = 5.5

The tissue culture plants grown from the tissue culture material were planted out on an expanded polystyrene tray. During the first 2 weeks the plants stood at 22-23°C and at high relative humidity (where appropriate, the plants may be left under a mist for the first two weeks). After the 2 weeks taking time they were left at 16-17°C in order to prevent strong protracted growth.

Although the availability of tissue culture material is not seasonally limited, the bloom induction period which extends from about mid-August to mid-October represents a restriction. The planting-out period therefore extends from beginning of December to end of June.

The tissue culture cuttings differ from normal (traditional) cuttings in that they are already branched by themselves and frequently possess two branches. The flower bud induction is seasonally limited just as with the normal (traditional) cutting. Since growth of tissue culture plants is fairly controlled they can best be grown in the cold greenhouse, in contrast to the traditionally cultured plants. In the first instance, a minimum night-time temperature of 15°C is maintained in this connection. This is allowed to drop to 10°C in September and to 5°C or even lower later on. This low temperature is needed to induce shedding of leaves.

The induction of flower buds of hydrangeas depends on a fairly complicated interaction between temperature, daylight, light intensity and shoot height and furthermore also depends on the variety. As mentioned, the natural flower bud formation of hydrangeas takes place in Europe from mid-August to mid-October. However, the optimum temperature for this flower bud formation is between 15 and 18°C during 6-11 weeks for most cultivated varieties.

When growth of the hydrangea has stopped and the flower buds are almost formed the stalk will begin to lignify. The leaf only dies off when the buds are fully developed. All the reserve nutrients from the old leaf are passed to the buds. As mentioned, a low temperature (5°C) promotes dying-off of the leaf, but the flower buds must have formed by then. A hydrangea is sensitive to frost until the stalks are really lignified and the flower buds completely closed. Freezing while there is still foliage on the plant or the epidermis cells are still green may possibly result in sites of mould infestation which do not manifest themselves until blossoms develop.

After the hydrangeas which have lignified and are provided with flower bud formation have spent the dormant period in a cold store dormancy may be broken by placing the plants in the warmth. The time required for forcing the hydrangeas into bloom is called the forcing period. For the tissue culture plants according to the invention, which were frozen, for example, this forcing period may be as little as 40 days in the summer months.

Normally, the forcing temperature for the hydrangeas until the buds come out is 22-24°C at a relative humidity of 80-90%. Once they have come out a temperature of 18-20°C is sufficient. The plants blossom earlier at higher temperature but growth takes longer.

Exemplary embodiment

The tissue culture plants obtained from the above-described tissue culture material were planted out in the following months of December to April.

The flower bud formation and lignification of the hydrangea plants took place in the period from September to October.

The lignified hydrangeas were stored in a cold store at a temperature of -2°C in the months of December and January. After a dormant period of at least 2 months the hydrangeas were forced into bloom over a period of 2-10 months. When the bloom period was later the dormant period of a minimum of 2 months of course had to be lengthened correspondingly. As soon as the plants came out of the cold they were immediately forced into bloom (forcing period 6-12 weeks). The test varieties were "Bodensee", "Masja" and "Wit".

Shortly before the freeze-treatment the plants were furthermore sprayed with the commercial product Rovral™* (normal metering). The result as regards forcing into bloom of the frozen, lignified hydrangeas was about 95%.

**Claims**

1. Method for forcing hydrangea plants (Hydrangea macrophylla (Thunb.) Ser.) into bloom after application of a dormant period for the hydrangea plants which are lignified and possess flower buds at reduced temperature, characterized in that hydrangea plants grown from tissue culture material are used which have undergone a dormant period at a temperature below 0°C after flower bud formation and lignification.

2. Method according to Claim 1, characterized in that hydrangea plants grown from tissue culture material are used which have undergone a dormant period at a temperature of 0 to -5°C.

3. Method according to Claim 2, characterized in that hydrangea plants grown from tissue culture material are used which have undergone a dormant period at a temperature of -0.5°C to -2°C.

4. Method according to one or more of Claims 1-3, characterized in that the dormant period covers a period of 1-12 months.

5. Method according to Claim 4, characterized in that the dormant period covers a period of 6-12 months.

6. Method for storing lignified hydrangea plants possessing flower buds at reduced temperature, characterized in that hydrangea plants grown from tissue culture material are stored at a temperature below 0°C after flower bud formation and lignification.

* active substance "Iprodione" (Chemical Pharmaceutical Industry Luxan, Netherlands)

EP 0 325 816 B1

7. Method according to Claim 6, characterized in that the hydrangea plants grown from tissue culture material are stored at a temperature of 0°C to -5°C.

8. Method according to Claim 7, characterized in that the hydrangea plants grown from tissue culture material are stored at a temperature of -0.5°C to -2°C.


## Patentansprüche

1. Verfahren, um Hortensienpflanzen (Hydrangea macrophylla (Thunb.) Ser.) zum Blühen zu bringen, nachdem die Hortensienpflanzen, die lignifiziert sind und Blütenknospen besitzen, bei verminderter Temperatur einer Ruheperiode unterworfen wurden, dadurch gekennzeichnet, daß aus Gewebekulturmaterial gezogene Hortensienpflanzen verwandt werden, die eine Ruheperiode bei einer Temperatur unterhalb 0°C nach der Blütenknospenbildung und Lignifizierung eingegangen sind.

2. Verfahren nach Anspruch 1, dadurch gekennzeichnet, daß aus Gewebekulturmaterial gezogene Hortensienpflanzen verwandt werden, die eine Ruheperiode bei einer Temperatur von 0 bis -5°C eingegangen sind.

3. Verfahren nach Anspruch 2, dadurch gekennzeichnet, daß aus Gewebekulturmaterial gezogene Hortensienpflanzen verwandt werden, die eine Ruheperiode bei einer Temperatur von -0,5°C bis -2°C eingegangen sind.

4. Verfahren nach einem oder mehreren der Ansprüche 1 bis 3, dadurch gekennzeichnet, daß die Ruheperiode einen Zeitraum von 1 bis 12 Monaten abdeckt.

5. Verfahren nach Anspruch 4, dadurch gekennzeichnet, daß die Ruheperiode einen Zeitraum von 6 bis 12 Monaten abdeckt.

6. Verfahren zur Lagerung lignifizierter Hortensienpflanzen mit Blütenknospen bei verminderter Temperatur, dadurch gekennzeichnet, daß aus Gewebekulturmaterial gezogene Hortensienpflanzen nach der Knospenbildung und Lignifizierung bei einer Temperatur von unter 0°C gelagert werden.

7. Verfahren nach Anspruch 6, dadurch gekennzeichnet, daß die aus Gewebekulturmaterial gezogenen Hortensienpflanzen bei einer Temperatur von 0°C bis -5°C gelagert werden.

8. Verfahren nach Anspruch 7, dadurch gekennzeichnet, daß aus Gewebekulturmaterial gezogene Hortensienpflanzen bei einer Temperätur von -0,5°C bis -2°C gelagert werden.


## Revendications

1. Procédé pour faire fleurir des hortensias (Hydrangea macrophylla (Thunb.) Ser.) après application d'une période de repos à température réduite aux hortensias qui sont lignifiés et possèdent des boutons floraux, caractérisé en ce que l'on utilise des hortensias ayant poussé à partir d'un produit de culture cellulaire qui ont subi une période de repos à une température inférieure à 0°C après formation de boutons floraux et lignification.

2. Procédé selon la revendication 1, caractérisé en ce que l'on utilise des hortensias ayant poussé à partir d'un produit de culture cellulaire qui ont subi une période de repos à une température de 0 à -5°C.

3. Procédé selon la revendication 2, caractérisé en ce que l'on utilise des hortensias ayant poussé à partir d'un produit de culture cellulaire qui ont subi une période de repos à une température de -0,5°C à -2°C.

4. Procédé selon l'une ou plusieurs des revendications 1 à 3, caractérisé en ce que la période de repos s'étend sur une période de 1 à 12 mois.

5. Procédé selon la revendication 4, caractérisé en ce que la période de repos s'étend sur une période de 6 à 12 mois.

6. Procédé pour conserver à température réduite des hortensias lignifiés possédant des boutons floraux, caractérisé en ce que des hortensias ayant poussé à partir d'un produit de culture cellulaire sont conservés à une température inférieure à 0°C après formation de boutons floraux et lignification.

7. Procédé selon la revendication 6, caractérisé en ce que les hortensias ayant poussé à partir d'un produit de culture cellulaire sont conservés à une température de 0°C à -5°C.

8. Procédé selon la revendication 7, caractérisé en ce que les hortensias ayant poussé à partir d'un produit de culture cellulaire sont conservés à une température de -0,5°C à -2°C.

5